# EUROPEAN PATENT SPECIFICATION

(11) **EP 3 378 474 B1**
(45) Date of publication and mention of the grant of the patent: **16.09.2020**
(21) Application number: 18162313.3
(22) Date of filing: 16.03.2018
(51) Int. Cl.: A61K 31/192, A61K 31/355, A61K 33/12, A61K 36/61, A61K 9/00, A61K 47/02, A61K 9/70, A61P 31/10, A61K 47/06, A61K 47/10, A61K 47/12, A61K 47/18, A61K 47/22, A61K 47/26, A61K 47/32, A61K 47/36, A61K 9/10, A61P 25/00

(54) **COMPOSITION FOR THE USE IN THE TREATMENT OF PLANTAR HYPERHIDROSIS PREDISPOSING TO CUTANEOUS FUNGAL INFECTION OF THE FOOT**
ZUSAMMENSETZUNG ZUR VERWENDUNG BEI DER BEHANDLUNG VON PLANTARER HYPERHIDROSE, DIE FÜR DIE KUTANE PILZINFEKTION DES FUSSES PRÄDISPONIEREND IST
COMPOSITION POUR UTILISATION DANS LE TRAITEMENT DE L'HYPERHIDROSE PLANTAIRE PRÉDISPOSANT À UNE INFECTION FONGIQUE CUTANÉE DU PIED

(30) Priority: 20.03.2017 IT 201700030588
(43) Date of publication of application: 26.09.2018
(73) Proprietor: Livieri, Federica, 60019 Senigallia (AN) (IT); Angeletti, Barbara, 61037 Mondolfo (PU) (IT)
(72) Inventor: LIVIERI, Federica, I-60019 Senigallia (AN) (IT); CAVALLO, Giovanni, I-00122 Ostia (RM) (IT); MORSIANI, Andrea, I-61032 Fano (PU) (IT)
(74) Representative: Sarpi, Maurizio

(56) References cited:
- WO-A1-2016/149376
- US-A1- 2001 005 510
- DATABASE WPI Week 201616 Thomson Scientific, London, GB; AN 2015-81081U XP002775459, & CN 105 078 793 A (UNIV SOUTH CHINA TECHNOLOGY) 25 November 2015 (2015-11-25)
- BUCK D S ET AL: "Comparison of two topical preparations for the treatment of onychomycosis: Melaleuca alternifolia (tea tree) oil and clotrimazole", JOURNAL OF FAMILY PRACTICE, NEW YORK, NY, US, vol. 38, no. 6, 31 May 1994 (1994-05-31), pages 601-605, XP009501433, ISSN: 0094-3509
- NIDHI AGGARWAL ET AL: "Preparation and Evaluation of Dermal Delivery System of Griseofulvin Containing Vitamin E-TPGS as Penetration Enhancer", AAPS PHARMSCITECH, SPRINGER NEW YORK LLC, US, vol. 13, no. 1, 1 December 2011 (2011-12-01), pages 67-74, XP035014941, ISSN: 1530-9932, DOI: 10.1208/S12249-011-9722-Y

## Description

### Field of application

In its most general aspect, the present invention relates to topical products for the reduction and elimination of plantar hyperhidrosis as a predisposing condition to fungal and microbial infections of the skin and of the nail, more particularly it concerns a composition for the prevention of superficial mycoses, of the skin and of the nail, in the presence of, and favored by, hyperhidrosis and the relative method of use allowing a significant beneficial effect on the patient's nail lasting over time.

### Background of the invention

Several studies have shown that the cutaneous mycosis of the foot and onychomycosis, i.e. the infection of the nail and the surrounding tissue supported by some species of fungi, molds and yeasts, affect around 30-50% of people; it is not just an aesthetic problem, but a real pathological condition, difficult to eradicate.

Onychomycosis can affect all the nails of the hands and feet, but those of the foot are subject to infection with a frequency twenty-five times higher than the fingernails; the toe is the most involved finger as it is the one most forced to withstand pressure and traumas of walking. The mycosis can affect any part of the nail that, once sick, undergoes an alteration of its natural color, it can become yellow or brown, moreover it appears more fragile, friable, deformed, and, sometimes, considerably thickened. Untreated infection, over time, can spread to other nails.

The factors predisposing to this pathology are numerous: aging (as the peripheral blood circulation slows down, consequently decreases the immune response and nail growth), familiarity, general or local pathological conditions (diabetes, peripheral vascular insufficiency, immunodepression, hyperhidrosis, tinea pedis, podological abnormalities). Environmental, professional and behavioral factors also increase the chances of contracting an infection, for example, more and more people are practicing sport using occlusive shoes and therefore not suitable for foot health, frequenting environments with warm-humid microclimate such as gym, swimming pools and wellness centers. Despite the improvement of personal hygiene and the conditions of the living environment, foot mycosis continues to spread and persist.

Since onychomycosis is a difficult condition to treat, taking a long time to completely solve the problem, and tending to recur over time, especially in predisposed subjects, becoming chronic, when the presence is suspected or the first clinical signs appear, these should not be overlooked, hoping they spontaneously disappear. Rather, it is advisable to undertake an appropriate antifungal therapy, having the compliance to follow it scrupulously for the necessary time.

A good prophylaxis and means of prevention are certainly one way to reduce the risk of infection in predisposed subjects.

Following a correct hygiene of the hands and feet is undoubtedly the best method to prevent the onset of mycoses; also it is advisable to wear shoes guaranteeing good ventilation and properly absorbing sweat, keep the nails short, disinfect the tools used in cleaning the nails, wash thoroughly hands after touching the infected nail, do not apply nail polish on the infected nail. General prevention guidelines also establish to act on favoring environmental factors, reducing the frequency of washing and the use of footwear and occlusive clothing, correcting any hyperhidrosis and maceration.

It has been observed that the total risk of any fungal cutaneous infection is increased in body sites with hyperhidrosis [Walling HW "Primary-to-heart disease increases the risk of cutaneous infection: a case-control study of 387 patients." J Am Acad Dermatol 2009. 61: 242-6]. In subjects suffering onychomycosis and hyperhidrosis skin maceration decreases defense against fungal infection and environmental humidity promotes the growth and proliferation of fungi. In a study by Zheng et al., out of 40 cases of patients presenting both onychomycosis and hyperhidrosis, 20 have been treated for onychomycosis, and the other 20 for both onychomycosis and hyperhidrosis Sixteen of the 20 patients treated for only onychomycosis healed, while all 20 patients treated for both conditions healed [Zheng et al. "Analysis of the factors influencing the therapeutic effects of onychomycosis". J Tongji Med Univ. 2001. 21: 259-62]. A German study revealed that the 30 patients affected by tinea pedis enrolled had a 3.5-fold higher hyperhidrosis level than patients without tinea pedis. Hyperhidrosis is the condition characterized by excessive sweating, higher than that required for normal thermoregulation, usually begins during childhood or adolescence. The disorder is diagnosed when sweating occurs in conditions where it is not normally expected, or excessively in response to emotional or thermal stimuli. This condition can be idiopathic (also known as primary or essential hyperhidrosis) or secondary to other diseases, such as metabolic disorders, febrile illness, or due to the use of drugs. The cases of primary hyperhidrosis are much more frequent than the secondary one and are generally localized to the hands, feet, underarms, or a combination of these. Tension and anxiety can provoke or aggravate perspiration, but psychological and/or psychiatric disorders are rarely the cause of the phenomenon. In general, hyperhidrosis affects about 3% of the population and includes people of both sexes and all races [Dermatologic Manifestations of the Lower Extremity, An Issue of Clinics in Podiatric Medicine and Surgery edited by Tracey C. Vlahovic, 2016; Cheshire W.P. and Freeman R. Disorders of Sweating. Semin Neurol. 2003. 23: 399-406], but surely it is an underestimated value not including the many cases not reported due to a certain embarrassment of the patient for the condition.

In cases where no specific physiological cause has been identified, current treatments for hyperhidrosis are symptomatic. In patients with primary hyperhidrosis, or for symptomatic treatment of excessive sweating in patients with secondary hyperhidrosis, otherwise untreatable, currently available treatments include local injections of botulinum toxin, surgical removal of sweat glands, topical deodorants containing aluminum, systemic use of anti-cholinergic drugs and treatment with electric currents.

Botulinum toxin injections have shown some effectiveness in the treatment of hyperhidrosis due to their anti-cholinergic effects at the neuromuscular junction and in the junctions between sympathetic postganglionic fibers and glands sweat. U.S. Patent No. 6,683,049 relates to a method of treatment of excessive sweating with botulinum toxin injections. The commercial product BOTOX® is indicated for the treatment of severe primary axillary hyperhidrosis, but it is practically useless for the treatment of excessive sweating in other parts of the body, including the palms of the hands and soles of the feet.

Sedatives and/or anti-cholinergic drugs are effective in reducing sweating, but the dosages required to reduce sweating can also cause side effects including dry mouth, constipation, blurred vision, decreased sexual ability, lack of appetite, nausea, drowsiness, sensation of temperature increase, among the most common. However, most patients with localized or generalized hyperhidrosis cannot tolerate them for long periods.

Another treatment option is ionophoresis, which requires the application of a low-intensity electrical current (15-18 mA) applied to the palms of the hands and/or the soles of the feet immersed in an electrolyte solution. Many are the disadvantages related to the application of this technique and include the need for repeated treatments, high cost, repetition of sweating after treatment interruption, difficulty in applying the treatment to the axillary region, and impracticability in the treatment of diffuse hyperhidrosis. Side effects include burning, feeling of electric shock, tingling and skin irritation. Iontophoresis can also be carried out in the presence of pharmacological therapeutic agents which are conveyed in the application site by ionophoretic procedure. In the international patent application No. WO 00/54834 a sweating control system which exploits the distribution by ionophoresis of an antiperspirant agent in the affected body regions is described. The surgical removal of sweat glands for primary hyperhidrosis is an alternative treatment that can provide some relief to the subject, but it can also produce unwanted side effects, including compensatory sweating.

Also hypnosis and laser therapy are other treatment options enabling some relief to the subject suffering from hyperhidrosis, but are expensive solutions for the patient.

Among the pharmaceutical-type solutions, US Patent 5,730,964 describes a method for treating conditions related to excessive sweating comprising the oral or topical administration of a therapeutically effective amount of an α-5-reductase inhibitor.

US Patent 6,433,003 describes a method for the treatment of hyperhidrosis comprising topical administration of a composition comprising from 0.25% to 6% of a glycopyrrolate compound.

US Patent No. 5,258,388 describes novel anticholinergic/anti-secretory agents useful as antiperspirants.

US Patent Application No. 20040192754 provides methods for the treatment of idiopathic hyperhidrosis comprising administering to a patient compounds reducing the activity of a 5-HT2C receptor. The composition comprising the 5-HT2C receptor antagonist may be administered concomitantly with antiperspirants, tranquilizers and anti-colinergic agents.

International patent application WO 2004/040660 describes compositions for the treatment of skin wrinkles and hyperhidrosis comprising components inhibiting acetylcholine release.

However, it is clear that the possible effects of these treatments, although applied to solve a localized problem, as in the case of plantar hyperhidrosis, can have important consequences at a generalized level in the whole organism.

Very common topical treatments contain aluminum derivatives, in particular aluminum soluble salts which are absorbed by the skin and act as energizing astringents and antisudorifers. The most commonly used are aluminum boroformate (3-5%), aluminum sulphate (2%, if used in a more concentrated form can have a caustic and irritating effect), aluminum phenolsulfate. However, aluminum-derived compounds efficacy is controversial as it produce skin irritation [Goh et al. Int. J. Dermatol. 1990. 29: 638-70; Holze et al. Dermatological 1987. 175: 126-135].

The Chinese patent CN 105 078 793 discloses a gel wash with antibacterial and anti-Candida activity comprising 6g of Tea tree oil, 100 mg of vitamin E TPGS and mandelic acid 0,5 g. Indeed, according to the description the antibacterial and anti-Candida gel has the following formulation: tea tree oil 2-10 %, chlorexidine acetate 0.5-8 %, water miscible vitamin E derivate 0.01-1%, skin protectant 0.5-10%, surfactant 0.5-2%, alcohol material 5-30 %, carbomer 0.5-4 %, pH regulator 0.5-4 %, deionized water; moreover in the document is specified that the mandelic acid is counted as pH regulator in the group of several pH regulators all equally working and selectable by the skilled person, so that according to the description it is possible to think to replace mandelic acid with any pH regulator of the group. No specific biocide action or contribution against *Candida albicans* strain is attributed to the mandelic acid.

Buck D.S. et al. in "Comparison of two topical preparation for the treatment of onychomycosis: Melaleuca alternifolia (tea tree) oil and clotrimazole". The Journal of family practice 199438: 601-605, discloses the antifungal activity of *Melaleuca alternifolia.*

US patent application 2001/0005510 discloses an anti-fungal treatment, for use on skin, comprising an anti-fungal component and a skin treating component. The skin treating component either absorbs moisture to create an undesirable environment for the fungus, or treats the skin to allow the anti-fungal component to penetrate, allow the skin to heal, and to create an unpleasant, oily environment for the fungus. The anti-fungal component is a combination of goldenseal root powder with ipe roxo powder and poke root powder. The skin treating component is either a skin softening mixture, such as D-alpha tocopherol and olive oil, or is a dehydrator, such as arrow root and ball clay. According to the invention a further combined anti-fungal and aromatic component preferably comprises lavender oil, and may also comprise tea tree oil.

Nidhi Aggarwal et al. describes preparation and evaluation of dermal delivery system of griseofulvin based on vitamin E-TPGS.

Despite the many types of approach to the problem of plantar hyperhidrosis, it is clear that the optimal therapeutic choice should be on topical, non-invasive treatment, which excludes undesirable side effects, easy to be administered and convenient for the patient. Therefore, the need of having alternative methods of treatment of hyperhidrosis and of compositions alternative to those of the state of the art is strongly felt, namely method of treatment of plantar hyperhidrosis as a predisposing condition to fungal infections of the foot and onychomycosis.

### Summary of the invention

The invention is defined by the claims. Any subject-matter falling outside the scope of the claims is provided by information purposes only. Furthermore, any references in the description to methods of treatment refer to the compounds, pharmaceutical compositions and medicaments of the present invention for use in a method of treatment of the human or animal body by therapy.

The present invention provides a composition for topical use in the treatment of plantar hyperhidrosis as a predisposing condition to the cutaneous mycosis of the foot, said composition includes the association of:
a) *Melaleuca alternifolia* oil extract (also known as tea tree oil),
b) mandelic acid, e
c) α-Tocopherol polyethylene glycol succinate,
in the presence of at least one hygroscopic agent.

A further object of the invention is the method based on the use of the composition comprising the combination of *Melaleuca alternifolia* oil extract, mandelic acid and α-Tocopherol polyethylene glycol succinate and at least one hygroscopic agent for treating plantar hyperhidrosis as a predisposing condition to the onset of cutaneous fungal infections of the foot.

### Brief description of the drawings

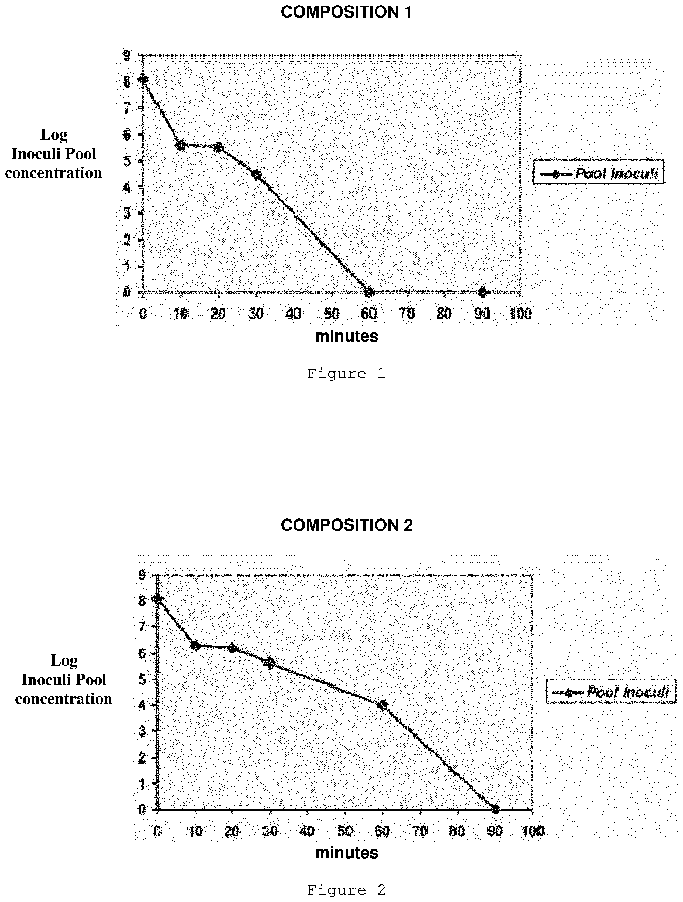

Figure 1 shows the logarithmic diagram of the inoculum pool concentration including dermatophyte fungal and *Candida albicans* strains in sample 1 comprising the combination of *Melaleuca alternifolia* oil extract, mandelic acid and α-Tocopherol polyethylene glycol succinate.
Figure 2 shows the logarithmic diagram of the inoculum pool concentration including dermatophyte fungal and *Candida albicans* strains in sample 2 comprising *Melaleuca alternifolia* oil extract.

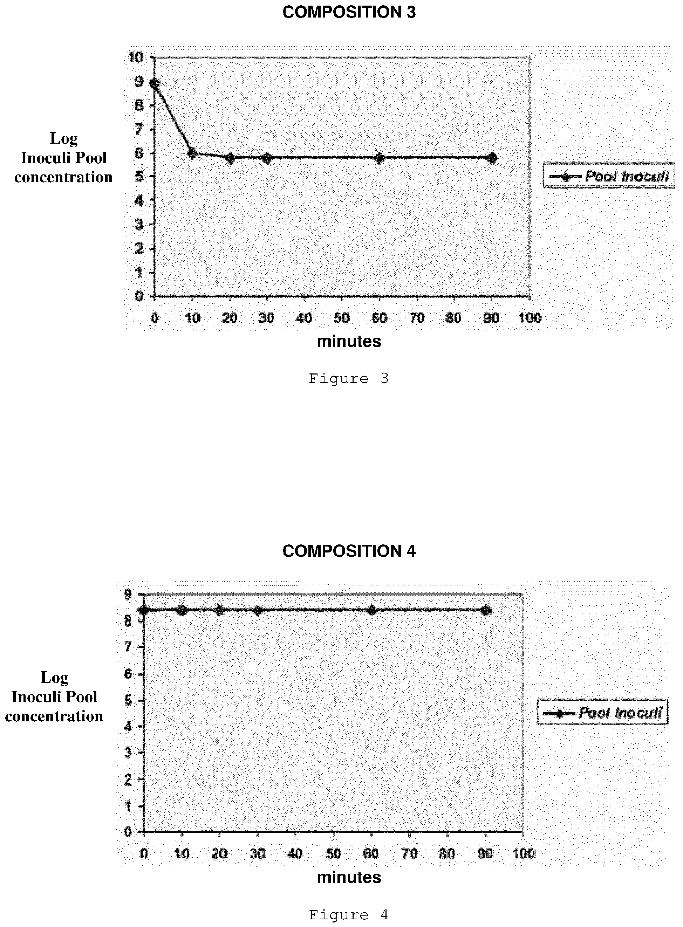

Figure 3 shows the logarithmic diagram of the inoculum pool concentration including dermatophyte fungal and *Candida albicans* strains in sample 3 comprising mandelic acid.
Figure 4 shows the logarithmic diagram of inoculum pool concentration including dermatophyte fungal and *Candida albicans* strains in sample 4 comprising α-Tocopherol polyethylene glycol succinate.

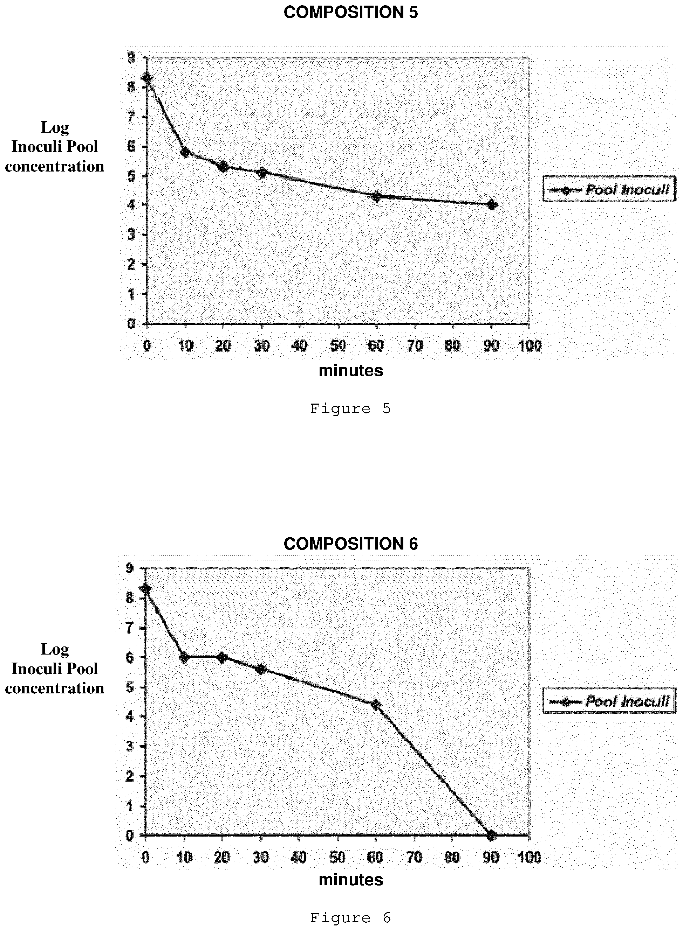

Figure 5 shows the logarithmic diagram of the inoculum pool concentration comprising dermatophyte fungal and *Candida albicans* strains in sample 5 comprising the association of *Melaleuca alternifolia* oil extract and mandelic acid.
Figure 6 shows the logarithmic diagram of inoculum pool concentration including dermatophyte fungal and *Candida albicans* strains in sample 6 comprising the association of *Melaleuca alternifolia* oil extract and α-Tocopherol polyethylene glycol succinate.

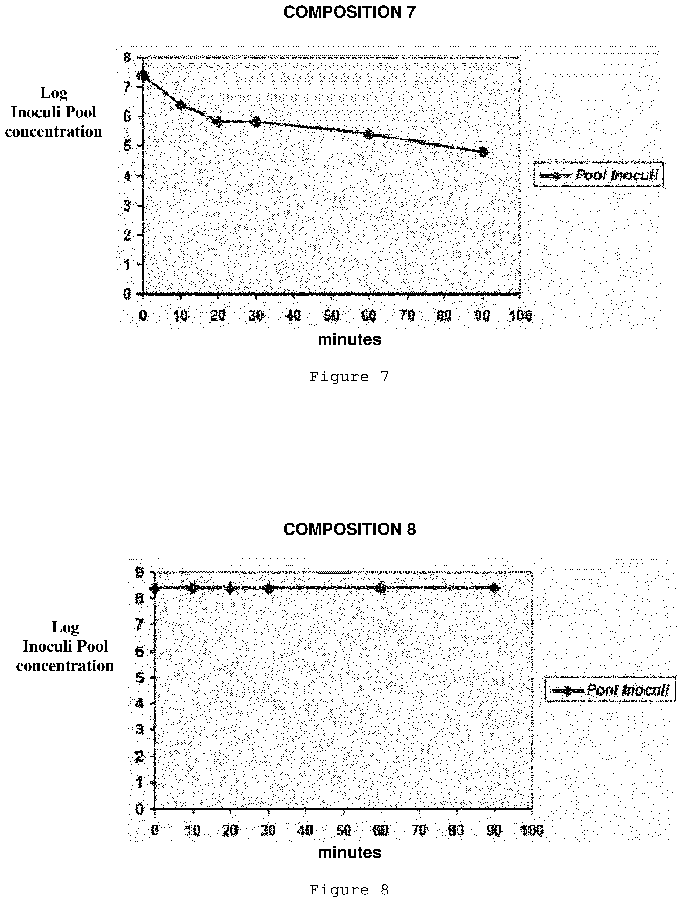

Figure 7 shows the logarithmic diagram of the inoculum pool concentration including dermatophyte fungal and *Candida albicans* strains in sample 7 comprising the association of mandelic acid and α-Tocopherol polyethylene glycol succinate.
Figure 8 shows the logarithmic diagram of inoculum pool concentration including dermatophyte fungal and *Candida albicans* strains in sample 8 of the negative control, devoid of the association and any components thereof.

### Detailed description of the invention

The aim of the present invention is to provide a topical composition for the treatment of plantar hyperhidrosis favouring the onset of cutaneous mycosis comprising the association of:
a) *Melaleuca alternifolia* oil extract (tea tree oil),
b) mandelic acid,
c) α-Tocopherol polyethylene glycol succinate (Vit. E TPGS)
in the presence of an hygroscopic agent.

The antifungal and antibacterial properties of the *Melaleuca alternifolia* oil extract are known; it can be used to disinfect the skin, has a mild antibiotic effect for external use, counteracts acne, carbuncles and all common skin pimples, it is a bland remedy against fleas and lice infestations. Few drops of essential tea tree oil can be used directly on the skin, or diluted, as antifungal agent to combat skin fungus, nail fungus, athlete's foot phenomenon, however the administration of topical products containing tea tree oil, while causing a significant improvement in the appearance of the treated skin and nail, determines a temporary aesthetic effect, where the remission of the infection is not confirmed by the microbiological analysis data, within a few weeks/months the nail returns assuming the typical aspect of the mycotic nail, the nail appears thick, fragile, friable or jagged, deformed, opaque, yellowed or dark in color, sign of the onset of a new infection or the recurrence of the previous one. Therefore, the administration of topical products containing tea tree oil as an active ingredient, likely for its fungistatic activity, rather than fungicide, is not useful for the treatment and prevention of recurrent onychomycosis.

The mandelic acid, belonging to the category of alpha-hydroxy acids, is a compound widely used in cosmetics for the formulation of exfoliating, lightening and antioxidants creams; it is indicated as bacteriostatic and antiseptic agent, is a valid active ingredient for a wide range of skin problems: it treats acne and wrinkles, in adults it is also effective in the treatment of severe acne, produces excellent results in the treatment of inflammatory, comedonic and papular pustular acne; the administration of mandelic acid has proved to be effective even in cases of acne wherein other types of medicines, or even antibiotics, have failed. The results may be visible as early as a few days after starting the treatment. It has been observed that a constant and gradual use of mandelic acid leads to significant improvements in the case of skin pigmentation abnormalities, the improvements have been recorded in particular for cases of: melasma, post-inflammatory hyperpigmentation and freckles. The mandelic acid also shares the same effectiveness of glycolic acid on wrinkles and fine lines of the skin, the texture of the skin visibly improves few weeks after the beginning of treatment based on mandelic acid. One of the most important actions carried out by mandelic acid is the skin renewal: in addition to having an antibacterial action, the acid works on the skin by breaking the link between dead and damaged cells still present on the skin and increasing the rate of cell regrowth.

The third ingredient of the association according to the present invention is α-Tocopherol polyethylene glycol succinate, a natural liposoluble form of vitamin E, rendered soluble in water by binding to a chemical agent called polyethylene glycol. This synthetic form of vitamin E has been developed to be more easily absorbed in the intestines of children having difficulty in absorbing fats and vitamin E from the diet, so increasing vitamin E levels in the blood and helping to prevent disorders of the nervous system due to vitamin E deficiency. In cosmetics and pharmaceuticals, tocofersolan is used, similarly to liposoluble natural vitamin E, for its antioxidant properties.

No antifungal and antibacterial properties have been reported in relation to vitamin E, nor to its water-soluble form, as also confirmed by the result of the experimentation conducted by the Applicants and presented below in the experimental section of this description.

Surprisingly it has been found that the *Melaleuca alternifolia* oil extract, in association with mandelic acid and α-Tocopherol polyethylene glycol succinate, in a topical composition comprising said association, exerts a much more effective and quicker antifungal action against a pool of microorganisms belonging to the species most frequently responsible for onychomycosis. In most of cases (about 85 to 90%) the agent responsible for the onychomycosis is a dermatophyte keratinophile fungus parasitizing the keratinized tissues such as the horny layer of the epidermis and the skin appendages (hair, hair and nails in humans and feathers, feathers, hairs, hooves, etc. in animals); those mainly involved are *Trichophyton rubrum* and *Trichophyton mentagrophytes;* more rarely, the disease is due to yeasts and non-dermatophyte moldes.

In the association according to the invention oil extract of *Melaleuca alternifolia,* mandelic acid and α-Tocopherol polyethylene glycol succinate are present respectively with a weight ratio: tea tree oil/mandelic acid/Vit. E TPGS 12/0.5/0.5. It has been observed that a composition comprising said association and at least one hygroscopic agent, .i.e. an agent capable of absorbing moisture from the surrounding environment, administered topically on the skin, is able to absorb excess moisture (hyperhidrosis) which in some subjects can promote the onset of skin infections having fungal or *Candida albicans* etiology.

The hygroscopic agent is a metal silicate, insoluble in water, but it hydrates and swells to form highly thixotropic colorless colloidal dispersions. According to the invention, the hygroscopic agent is laponite, a colloidal synthetic clay used as an additive in various products to modify the rheology thereof and to create films acting as antistatic barriers. The XLG laponite used in the composition according to the invention is a commercial product (Rockwood Additives), obtained from the combination of sodium, lithium and magnesium salts with sodium silicates at controlled temperatures (INCI name: Sodium Magnesium Silicate) consisting of anhydrous form SiO₂ (59.5%), MgO (27.5%), Li₂O (0.8%), Na₂O (2.8%). The cells that compose it have a characteristic disk shape. A cell consists of a layer of magnesium ions placed between two layers of silicon, all balanced by oxygen and hydroxyl atoms. During the dispersion process the crystals form aggregates held together by electrostatic bonds with the constitution of an electric double layer. In the presence of salts, the crystals assume a "card castle" structure, responsible for the thixotropic character of the gel. Laponite is used in many everyday products, such as cosmetics, toothpastes, detergents, paint thickeners.

According to the invention the hygroscopic agent is present in the composition in percentage ranging from 1 to 10% by weight of the total composition.

In the preferred embodiments of the invention the composition further comprises: carrier agents, solvents, essential oils, fragrances and perfumes, preservatives, emollient and moisturizing agents, antioxidant agents, surfactants, suspending agents, binders, filmogens and viscosers, thickeners, chelating agents, buffering agents, pharmaceutically accepted and known to those skilled in the art, so as to provide a topical formulation of easy and pleasant use by the end user. Therefore, in some particularly preferred embodiments, the composition can further comprise essential oils such as lavender oil, bergamot, eucalyptus, geraniol, carnosine, lipoic acid, propylene glycol, thickeners such as xanthan gum, hydroxyethyl acrylate/sodium acryloyl dimethyl taurate copolymer.

An object of the invention is also the use of the described composition comprising the association of:
a) *Melaleuca alternifolia* oil extract,
b) mandelic acid,
c) α-Tocopherol polyethylene glycol succinate,
in the treatment of plantar hyperhydrosis as predisposing condition to the foot cutaneous mycosis and onychomycosis.

Such use involves the application of an effective amount of the composition on the skin and on the foot nail once a day, generally in the evening before going to sleep, after cleansing; the composition is rubbed on nail and skin until completely absorbed. The treatment can also be prolonged in time since it is indicated as a prophylactic remedy useful to counteract in some subjects the typical conditions favoring colonization of pathogens responsible for cutaneous infections, namely onychomycosis.

The term "treatment", as used in the present description, means a reduction in the severity and/or frequency of symptoms, the elimination of symptoms and/or underlying cause, the prevention of the occurrence of symptoms and/or their cause, the improvement or remission of the condition.

Therefore, specifically the use of the present composition in a patient enables the prevention of the occurrence of foot cutaneous mycosis, evidentiated by a lengthening of the period of time between two fungal infection events in succession in the same area in a predisposed subject.

The term "topical administration" or "topical application" is herein used according to its conventional meaning to indicate that the composition of the invention is applied on the foot skin, on nail plate of the feet.

The term "therapeutically effective amount" means a non-toxic quantity, sufficient to provide the desired effect, that is, the reduction of the excess moisture on the skin of the feet, and ultimately the prevention of foot mycoses, as above mentioned. The "therapeutically effective amount" varies from subject to subject, depending on the general condition of the subject, the mode of administration, and the like. Therefore, it is not always possible to specify the exact extent of the "therapeutically effective amount". However, an appropriate "effective" amount in each individual case can be determined by the podiatric operator of ordinary skill in the art. Due to the high degree of penetrance of hyperhidrosis, the dosage regimen of the applied composition and the timing of administration are adapted according to the real needs of the subject receiving the treatment. This need is determined by clinical analysis to which the subject is subjected before starting treatment and at regular intervals during treatment to verify the effectiveness of the method, the good tolerability of the subject and the lack of side effects.

According to the present invention, the patient in the need of the treatment is a Mammal, preferably a Primate, even more preferably a human being.

Through the use of the composition for topical use as described, a synergistic effect is established between the draining action exerted by the hygroscopic agent and the antifungal action enabling to prevent fungal infection especially in those patients who, presenting excess moisture and maceration of the skin, provide an ideal substrate for the growth and proliferation of fungi and yeasts responsible for the infection.

The composition according to the invention comprising the association of:
a) *Melaleuca alternifolia* oil extract (tea tree oil),
b) mandelic acid,
c) α-Tocopherol polyethylene glycol succinate (Vit. E TPGS),
and at least one hygroscopic agent, is now illustrated by means of formulation examples of particularly preferred embodiments having no limitative intent, where all the quantities of the components are expressed as a percentage by weight of the composition.

### EXAMPLE 1

| Ingredient | Range % w/w | % w/w |
|---|---|---|
| *Melaleuca alternifolia* oil extract | 10-30 | 25 |
| Mandelic acid | 0.1-1 | 0.5 |
| Vit. E TPGS | 0.1-1 | 0.5 |
| Propylene glycol | 1-10 | 5.0 |
| Xanthan gum | 0.5-5 | 1.5 |
| Carnosine | 0.1-1 | 0.5 |
| Lipoic acid | 0.1-1 | 0.5 |
| EDTA Na₂H₂xH₂O | 0.01-2 | 0.20 |
| Laponite XGL | 1-10 | 10 |
| Deionized water q. s. to | | 100 |

### EXAMPLE 2

| Ingredient | % w/w |
|---|---|
| *Melaleuca alternifolia* oil extract | 12 |
| *Lavandula angustifolia* oil | 6 |
| Mandelic acid | 0.5 |
| Vit. E TPGS | 0.5 |
| Carnosine | 0.5 |
| Lipoic acid | 0.5 |
| Propylene glycol | 2.5 |
| EDTA Na₂H₂xH₂O | 0.20 |
| Xanthan gum | 2 |
| Laponite XGL | 5 |
| Deionized water q. s. to | 100 |

### EXAMPLE 3

| Ingredient | % w/w |
|---|---|
| *Melaleuca alternifolia* oil extract | 12 |
| *Lavandula angustifolia* oil | 6 |
| Mandelic acid | 0.5 |
| Vit. E TPGS | 0.5 |
| Carnosine | 0.5 |
| Lipoic acid | 0.5 |
| Propylene glycol | 2.5 |
| Tetrasodium glutamate diacetate | 0,20 |
| Xanthan gum | 1 |
| Sodium dehydroacetate | 0.20 |
| Simulgel NS (Copolymer hydroxyethyl acrylate/sodium acryloyldimethyl taurate, squalane, polysorbate 60) | 1.5 |
| Laponite XGL | 5 |
| Deionized water q. s. to | 100 |

### EXAMPLE 4

| Ingredient | % w/w |
|---|---|
| *Melaleuca alternifolia* oil extract | 12 |
| Mandelic acid | 0.5 |
| Vit. E TPGS | 0.5 |
| *Lavandula angustifolia* oil | 6.0 |
| Propylene glycol | 2.5 |
| Xanthan gum | 2.0 |
| Linalool | 1.5 |
| Hydroxyethyl acrylate/sodium acryloyl dimethyl taurate copolymer. | 1.11 |
| Squalane | 0.765 |
| Carnosine | 0.5 |
| Lipoic acid | 0.5 |
| Limonene | 0.204 |
| Polysorbate 60 | 0.165 |
| Tetrasodium glutamate diacetate | 0.094 |
| Sodium dehydroacetate | 0.2 |
| Geraniol | 0.06 |
| Sorbitan isostearate | 0.045 |
| Citronellol | 0.006 |
| Coumarine | 0.006 |
| Silicate of sodium, magnesium, lithium | 5.0 |
| Deionized water q. s. to | 100 |

### Conclusion

The present invention has demonstrated to overcome the technical problems still present in the methods of treating the plantar hyperhidrosis condition in subjects predisposed to fungal infections of the skin and the cutaneous appendages of the foot as it provides a topical, non-invasive treatment, effective, which does not cause skin irritation and completely free from unwanted side effects, easy to administer and convenient for the patient.

### Experimental part

*In vitro* evaluation of antifungal activity against fungi dermatophytes and yeasts.

The activity of the composition according to the invention was evaluated with respect to four of the most responsible species for fungal infection of the nail, in particular, three strains of dermatophyte fungi: *Trichophyton rubrum, Trichophyton mentagrophytes* and *Epidermophyton floccosum* and yeast *Candida albicans,* by comparing the efficacy of action of eight embodiments of the composition according to example 1 differing in the presence of one or two out the three elements of the association. In this way the test allowed to assess the contribution of each member of the association of *Melaleuca alternifolia* oil extract (tea tree oil), mandelic acid, α-Tocopherol polyethylene glycol succinate, and the synergic effect of the three ingredients in the determination of the antimicrobial activity against the tested species.

The activity was evaluated by means of viability test of the tested strains by counting the remaining vital colonies after incubation of dilutions of known titre of the four pooled strains in Sabouraud Dextrose Agar plates at 22-25 °C for 5 days.

Tested strains: *Trichophyton mentagrophytes* ATCC 9533
*Trichophyton rubrum* IHEM 02770,
*Epidermophyton floccosum* ATCC 52066,
*Candida albicans* ATCC 10231.

### Tested compositions according to the invention:

Sample 1: formulation as described in Example 1. *Lavandula angustifolia* oil, Propylene glycol, Xanthan gum, Linalool, Hydroxyethyl acrylate / sodium acryloyl dimethyl taurate copolymer, Squalane, Carnosine, Lipoic acid, Limonene, Polysorbate 60, Tetrasodium glutamate diacetate, Geraniol , Sorbitan isostearate, Citronellol, Coumarine, Deionized water and **association of *Melaleuca alternifolia* oil extract, mandelic acid and α-Tocopherol polyethylene glycol succinate.**
Sample 2: *Lavandula angustifolia* oil, Propylene glycol, Xanthan gum, Linalool, Hydroxyethyl acrylate/sodium acryloyl dimethyl taurate copolymer, Squalane, Carnosine, Lipoic acid, Limonene, Polysorbate 60, Tetrasodium glutamate diacetate, Geraniol, Sorbitan isostearate, Citronellol, Coumarine, Deionized water and ***Melaleuca alternifolia* oil extract.**
Sample 3: *Lavandula angustifolia* oil, Propylene glycol, Xanthan gum, Linalool, Hydroxyethyl acrylate/sodium acryloyl dimethyl taurate copolymer, Squalane, Carnosine, Lipoic acid, Limonene, Polysorbate 60, Tetrasodium glutamate diacetate, Geraniol, Sorbitan isostearate, Citronellol, Coumarine, Deionized water and **mandelic acid.**
Sample 4: *Lavandula angustifolia* oil, Propylene glycol, Xanthan gum, Linalool, Hydroxyethyl acrylate/sodium acryloyl dimethyl taurate copolymer, Squalane, Carnosine, Lipoic acid, Limonene, Polysorbate 60, Tetrasodium glutamate diacetate, Geraniol, Sorbitan isostearate, Citronellol, Coumarine, Deionized water and **α-Tocopherol polyethylene glycol succinate.**
Sample 5: *Lavandula angustifolia* oil, Propylene glycol, Xanthan gum, Linalool, Hydroxyethyl acrylate/sodium acryloyl dimethyl taurate copolymer, Squalane, Carnosine, Lipoic acid, Limonene, Polysorbate 60, Tetrasodium glutamate diacetate, Geraniol, Sorbitan isostearate, Citronellol, Coumarine, Deionized water and **association of *Melaleuca alternifolia* oil extract and mandelic acid.** Sample 6: *Lavandula angustifolia* oil, Propylene glycol, Xanthan gum, Linalool, Hydroxyethyl acrylate/sodium acryloyl dimethyl taurate copolymer, Squalane, Carnosine, Lipoic acid, Limonene, Polysorbate 60, Tetrasodium glutamate diacetate, Geraniol, Sorbitan isostearate, Citronellol, Coumarine, Deionized water and **association of *Melaleuca alternifolia* oil extract and α-Tocopherol polyethylene glycol succinate.**
Sample 7: *Lavandula angustifolia* oil, Propylene glycol, Xanthan gum, Linalool, Hydroxyethyl acrylate/sodium acryloyl dimethyl taurate copolymer, Squalane, Carnosine, Lipoic acid, Limonene, Polysorbate 60, Tetrasodium glutamate diacetate, Geraniol, Sorbitan isostearate, Citronellol, Coumarine, Deionized water and **association of mandelic acid and α-Tocopherol polyethylene glycol succinate.**
Sample 8: (Negative Control) *Lavandula angustifolia* oil, Propylene glycol, Xanthan gum, Linalool, Hydroxyethyl acrylate/sodium acryloyl dimethyl taurate copolymer, Squalane, Carnosine, Lipoic acid, Limonene, Polysorbate 60, Tetrasodium glutamate diacetate, Geraniol, Sorbitan isostearate, Citronellol, Coumarine, Deionized water.

Preparation of the strains. The freeze-dried strains obtained from the manufacturer have been cultured in their growth media and incubated at optimal temperature. Preparation of suspensions of the tested strains having known titre. According to the procedure provided in the Italian Official Pharmacopoeia (FUI XII ed.) a stock-suspension having known concentration for each single strains: *Trichophyton mentagrophytes, Trichophyton rubrum, Epidermophyton floccosum* and *Candida albicans,* grown in soil Tryptic Soy Broth (Liofilchem Srl) was prepared. The stock suspensions for each strain were used to prepare a stock suspension of the pool suspension of known title.

Procedure. The pool of strain was inoculated into four plates with optimal growth media each containing the composition (sample 1-8) to be assessed for its antifungal activity. Colture samples were withdrawn after 10, 20, 30, 60, 90 minutes according to F.U.I. XII ed.

### Results

### Sample 1 - composition with association of Melaleuca alternifolia oil extract, mandelic acid and α-Tocopherol polyethylene glycol succinate (Example 1)

**Table 1 - Counts of living micro-organisms (cfu/ml)**

| | cfu/ml | Log |
|---|---|---|
| T0 (Initial pool concentration) | 13x10⁷ | 8.1 |
| T₁₀ₘ | 45x10⁴ | 5.6 |
| T₂₀ₘ | 31x10⁴ | 5.5 |
| T₃₀ₘ | 3x10⁴ | 4.5 |
| T₆₀ₘ | No recovery (NR) | NR |
| T₉₀ₘ | No recovery (NR) | NR |

**Table 2 - Logaritmic reduction of microrganisms number**

| | T₁₀ₘ | T₂₀ₘ | T₃₀ₘ | T₆₀ₘ | T₉₀ₘ |
|---|---|---|---|---|---|
| Inoculed pool: | 2.5 | 2.6 | 3.6 | NR | NR |
| *Trichophyton mentagrophytes ATCC 9533* | | | | | |
| *Trichophyton rubrum IHEM 02770,* | | | | | |
| *Epidermophyton floccosum ATCC 52066,* | | | | | |
| *Candida albicans ATCC 10231* | | | | | |

The results show that sample 1, corresponding to the composition according to the invention comprising the **association of *Melaleuca alternifolia* oil extract, mandelic acid and α-Tocopherol polyethylene glycol succinate** has totally reduced the presence of microorganisms in the pool consisting of *Trichophyton mentagrophytes, Trichophyton rubrum, Epidermophyton floccosum, Candida albicans* after 60 minutes of treatment.

### Sample 2 - composition with Melaleuca alternifolia oil extract.

**Table 3 - Counts of living micro-oranisms (cfu/ml)**

| | cfu/ml | Log |
|---|---|---|
| T0 (Initial pool concentration) | 13x10⁷ | 8.1 |
| T₁₀ₘ | 22x10⁵ | 6.3 |
| T₂₀ₘ | 17x10⁵ | 6.2 |
| T₃₀ₘ | 41x10⁴ | 5.6 |
| T₆₀ₘ | 97x10² | 4.0 |
| T₉₀ₘ | No recovery (NR) | NR |

**Table 4 - Logaritmic reduction of microrganisms number**

| | T₁₀ₘ | T₂₀ₘ | T₃₀ₘ | T₆₀ₘ | T₉₀ₘ |
|---|---|---|---|---|---|
| Inoculed pool: | 1.8 | 1.9 | 2.5 | 4.1 | NR |
| *Trichophyton mentagrophytes ATCC 9533* | | | | | |
| *Trichophyton rubrum IHEM 02770,* | | | | | |
| *Epidermophyton floccosum ATCC 52066,* | | | | | |
| *Candida albicans ATCC 10231* | | | | | |

The results show that sample 2 corresponding to the composition according to the invention comprising only ***Melaleuca alternifolia* oil extract,** has totally reduced the presence of microorganisms in the pool consisting of: *Trichophyton mentagrophytes, Trichophyton rubrum, Epidermophyton floccosum, Candida albicans* after 90 minutes of treatment.

### Sample 3 composition with mandelic acid

**Table 5 - Counts of living micro-organisms (cfu/ml)**

| | cfu/ml | Log |
|---|---|---|
| T0 (Initial pool concentration) | 87x10⁷ | 8.9 |
| T₁₀ₘ | 1x10⁶ | 6.0 |
| T₂₀ₘ | 7x10⁵ | 5.8 |
| T₃₀ₘ | 7x10⁵ | 5.8 |
| T₆₀ₘ | 7x10⁵ | 5.8 |
| T₉₀ₘ | 6x10⁵ | 5.8 |

**Table 6 - Logaritmic reduction of microrganisms number**

| | T₁₀ₘ | T₂₀ₘ | T₃₀ₘ | T₆₀ₘ | T₉₀ₘ |
|---|---|---|---|---|---|
| Inoculed pool: | 2.9 | 3.1 | 3.1 | 3.1 | 3.1 |
| *Trichophyton mentagrophytes ATCC 9533* | | | | | |
| *Trichophyton rubrum IHEM 02770,* | | | | | |
| *Epidermophyton floccosum ATCC 52066,* | | | | | |
| *Candida albicans ATCC 10231* | | | | | |

The results show that the sample 3 corresponding to the composition according to the invention comprising mandelic acid has totally reduced the concentration of microorganisms in the pool consisting of *Trichophyton mentagrophytes, Trichophyton rubrum, Epidermophyton floccosum,* and *Candida albicans* of 2.9 logaritmic units after 10 minutes of treatment. After 10 minutes and up to 90 minutes the composition has fungistatic activity.

### Sample 4 - composition with α-Tocopherol polyethylene glycol succinate

**Table 7 - Counts of living micro-organisms (cfu/ml)**

| | (cfu/ml) | Log |
|---|---|---|
| T0 (Initial pool concentration) | 26x10⁷ | 8.4 |
| T₁₀ₘ | 26x10⁷ | 8.4 |
| T₂₀ₘ | 26x10⁷ | 8.4 |
| T₃₀ₘ | 26x10⁷ | 8.4 |
| T₆₀ₘ | 25x10⁷ | 8.4 |
| T₉₀ₘ | 25x10⁷ | 8.4 |

**Table 8 - Logaritmic reduction of microrganisms number**

| | T₁₀ₘ | T₂₀ₘ | T₃₀ₘ | T₆₀ₘ | T₉₀ₘ |
|---|---|---|---|---|---|
| Inoculed pool: | 0 | 0 | 0 | 0 | 0 |
| *Trichophyton mentagrophytes ATCC 9533* | | | | | |
| *Trichophyton rubrum IHEM 02770,* | | | | | |
| *Epidermophyton floccosum ATCC 52066,* | | | | | |
| *Candida albicans ATCC 10231* | | | | | |

The results show that the sample 4 corresponding to the composition according to the invention comprising α-Tocopherol polyethylene glycol succinate has no fungicide activity.

### Sample 5 - composition with association of Melaleuca alternifolia oil extract and mandelic acid

**Table 9 - Counts of living micro-organisms (cfu/ml)**

| | cfu/ml | Log |
|---|---|---|
| T0 (Initial pool concentration) | 20x10⁷ | 8.3 |
| T₁₀ₘ | 64x10⁴ | 5.8 |
| T₂₀ₘ | 19x10⁴ | 5.3 |
| T₃₀ₘ | 12x10⁴ | 5.1 |
| T₆₀ₘ | 2x10⁴ | 4.3 |
| T₉₀ₘ | 1x10⁴ | 4.0 |

**Table 10 - Logaritmic reduction of microrganisms number**

| | T₁₀ₘ | T₂₀ₘ | T₃₀ₘ | T₆₀ₘ | T₉₀ₘ |
|---|---|---|---|---|---|
| Inoculed pool: | 2.5 | 3.0 | 3.2 | 4.0 | 4.3 |
| *Trichophyton mentagrophytes ATCC 9533* | | | | | |
| *Trichophyton rubrum IHEM 02770,* | | | | | |
| *Epidermophyton floccosum ATCC 52066,* | | | | | |
| *Candida albicans ATCC 10231* | | | | | |

The results show that sample 5, corresponding to the composition according to the invention comprising the **association of *Melaleuca alternifolia* oil extract and mandelic acid** has reduced the presence of microorganisms in the pool consisting of *Trichophyton mentagrophytes, Trichophyton rubrum, Epidermophyton floccosum, Candida albicans* of 2.5 logaritmic units, afterwards a gradual decrease up to 90 minutes treatment occurs.

### Sample 6 - composition with association of Melaleuca alternifolia oil extract and α-Tocopherol polyethylene glycol succinate

**Table 11 - Counts of living micro-organisms (cfu/ml)**

| | cfu/ml | Log |
|---|---|---|
| T0 (Initial pool concentration) | 20x10⁷ | 8.3 |
| T₁₀ₘ | 2x10⁶ | 6.0 |
| T₂₀ₘ | 1x10⁶ | 6.0 |
| T₃₀ₘ | 6x10⁵ | 5.6 |
| T₆₀ₘ | 3x10⁴ | 4.4 |
| T₉₀ₘ | NR | NR |

**Table 12 - Logaritmic reduction of microrganisms number**

| | T₁₀ₘ | T₂₀ₘ | T₃₀ₘ | T₆₀ₘ | T₉₀ₘ |
|---|---|---|---|---|---|
| Inoculed pool: | | | | | |
| *Trichophyton mentagrophytes ATCC 9533 Trichophyton rubrum IHEM 02770, Epidermophyton floccosum ATCC 52066, Candida albicans ATCC 10231* | 2.3 | 2.3 | 2.7 | 3.9 | NR |

The results show that sample 6, corresponding to the composition according to the invention comprising the **association of *Melaleuca alternifolia* oil extract and α-Tocopherol polyethylene glycol succinate** has totally reduced the presence of microorganisms in the pool consisting of *Trichophyton mentagrophytes, Trichophyton rubrum, Epidermophyton floccosum, Candida albicans* 90 minutes after treatment.

### Sample 7 - composition with association of mandelic acid and α-Tocopherol polyethylene glycol succinate

**Table 13 - Counts of living micro-organisms (cfu/ml)**

| | cfu/ml | Log |
|---|---|---|
| T0 (Initial pool concentration) | 23x10⁶ | 7.4 |
| T₁₀ₘ | 23x10⁵ | 6.4 |
| T₂₀ₘ | 67x10⁴ | 5.8 |
| T₃₀ₘ | 67x10⁴ | 5.8 |
| T₆₀ₘ | 26x10⁴ | 5.4 |
| T₉₀ₘ | 7x10⁴ | 4.8 |

**Table 14 - Logaritmic reduction of microrganisms number**

| | T₁₀ₘ | T₂₀ₘ | T₃₀ₘ | T₆₀ₘ | T₉₀ₘ |
|---|---|---|---|---|---|
| Inoculed pool: | | | | | |
| *Trichophyton mentagrophytes ATCC 9533 Trichophyton rubrum IHEM 02770, Epidermophyton floccosum ATCC 52066, Candida albicans ATCC 10231* | 1.0 | 1.6 | 1.6 | 2.0 | 2.6 |

The results show that sample 7, corresponding to the composition according to the invention comprising the **association of mandelic acid and α-Tocopherol polyethylene glycol succinate** has reduced of one logartimic unit the concentration of microorganisms in the pool consisting of: *Trichophyton mentagrophytes, Trichophyton rubrum, Epidermophyton floccosum, Candida albicans* and progressive gradual concentration reduction up to 90 minutes.

### Sample 8 - negative control composition without association components

**Table 15 - Counts of living micro-oranisms (cfu/ml)**

| | (cfu/ml) | Log |
|---|---|---|
| T0 (Initial pool concentration) | 25x10⁷ | 8.3 |
| T₁₀ₘ | 25x10⁷ | 8.3 |
| T₂₀ₘ | 25x10⁷ | 8.3 |
| T₃₀ₘ | 25x10⁷ | 8.3 |
| T₆₀ₘ | 25x10⁷ | 8.3 |
| T₉₀ₘ | 25x10⁷ | 8.3 |

**Table 16 - Logaritmic reduction of microrganisms number**

| | T₁₀ₘ | T₂₀ₘ | T₃₀ₘ | T₆₀ₘ | T₉₀ₘ |
|---|---|---|---|---|---|
| Inoculed pool: | | | | | |
| *Trichophyton mentagrophytes ATCC 9533 Trichophyton rubrum IHEM 02770, Epidermophyton floccosum ATCC 52066, Candida albicans ATCC 10231* | 0 | 0 | 0 | 0 | 0 |

The results show that sample 8 corresponding to the composition according to the invention without any association components has no fungicide activity.

The obtained results demonstrate that the association according to the invention is able to perform an *in vitro* very rapid fungicidal action against the species most responsible for the infection such as dermatophyte fungi: *Trichophyton rubrum, Trichophyton mentagrophytes, Epidermophyton floccosum* and *Candida albicans* yeast. Otherwise, the ingredients of the association, respectively oil extract of *Melaleuca alternifolia,* mandelic acid and α-Tocopherol polyethylene glycol succinate, when individually tested or have no antifungal activity, neither fungicidal, nor fungistatic, as in the case of α-Tocopherol polyethylene glycol succinate (composition 4), or have a limited ability to reduce the concentration of the inoculated microbial pool which is established in the first ten minutes after inoculation, and subsequently a constant fungistatic activity over time, which however does not lead to the total elimination of the infection causative agent, establishes. In the case of mandelic acid (composition 3), or, in the case of the oil extract of *Melaleuca alternifolia* (composition 2), after the initial fungicidal effect in the first ten minutes after the inoculum the fungistatic action is accompanied by a very slow fungicidal effect leading to an increase of 50% of the time necessary to have the total reduction of the concentration of the inoculum (90 minutes) compared to the time when the same effect is established on the same species with the association of the three compounds (60 minutes) (composition 1).

A further interesting observation on the synergistic effect of the ingredients of the association object of the invention is that when the α-Tocopherol polyethylene glycol succinate, without any antifungal activity itself, is added respectively to the composition comprising tea tree oil (composition 6) or to the composition comprising mandelic acid (composition 3) the fungicidal action does not substantially change, as shown by the logarithmic diagram profiles of the concentration of microorganisms in the inoculum pool (sample 2 vs. sample 6; sample 3 vs. sample 7). When, instead, the α-Tocopherol polyethylene glycol succinate as the third ingredient is added to the combination of tea tree oil and mandelic acid (composition 5) with fungistatic activity, it is observed the establishment of a very intense and rapid fungicidal action (sample 1 vs. sample 5), with a rapidity such as to reduce by 50% the total reduction time of the concentration of the inoculum pool, likely due to the action of tea tree oil (sample 1 vs. sample 2 and sample 6).

### Clinical study

An assessment of the efficacy of the composition and of the method according to the invention has been carried out on patients treated with the described composition having excessive sweating of the foot and incipient maceration of the skin of the foot wherein frequently recurs the condition of mycosis of the foot with involvement of the skin appendages (nails), due to dermatophyte and non-dermatophyte fungi.

Following an accurate clinical analysis, the diagnosis of onychomycosis was considered confirmed in the presence of positivity and microscopic examination of the culture, in consideration of the frequency of false negative (Arrese JE et al. Facing up to the diagnostic and management of uncertainty onychomycoses. Int. J. Dermatol 1999; 38, suppl.2, 1-6).

Patients enrolled in the study have received the treatment according to the methods of administration and dosing regimen provided by the method herein described.

Efficacy was measured in terms of decreased area affected by infection, decreased number of recurrent infections and lengthening of time between two consecutive infections in the same patient, confirmed by the microbiology data, compared to baseline (T0), at regular time intervals from the start of treatment and at the end of the treatment period (T12 months). The follow-up of the treated patients is currently ongoing, the observations and results collected so far are very encouraging; none of the treated subjects reported signs of skin irritation. Detailed analysis of the data obtained from the *in vivo* study will be available soon.

## Claims

1. A composition **characterized in that** it comprises the association of:
a) *Melaleuca alternifolia* oil extract,
b) mandelic acid,
c) α-Tocopherol polyethylene glycol succinate,
in the presence of at least an hygroscopic agent, wherein said three components of the association respectively are in weight ratio: tea tree oil/mandelic acid/Vit. E TPGS of 12/0.5/0.5 and the hygroscopic agent is a metal silicate between 1 and 10 % by total weigh of composition.

2. The composition according to claim 1, wherein the metal is sodium, lithium, magnesium.

3. The composition according to claims 1 and 2 wherein the hygroscopic agent is laponite.

4. The composition according to any one of the preceding claims further comprising: carrier agents, solvents, essential oils, fragrances and perfumes, preservatives, emollients and moisturizing agents, antioxidants, vitamins, surfactants, suspending agents, binders, film-forming and viscosity agents, thickeners, chelating agents, pharmaceutically accepted buffering agents.

5. The composition according to claim 4, wherein carriers agents, solvents, essential oils, fragrances and perfumes, preservatives, emollients and moisturizing agents, antioxidant agents, vitamins, surfactants, suspending agents, binders, film-forming and viscosity agents, thickeners, chelating agents, pharmaceutically accepted buffering agents, are: lavender oil, bergamot oil, eucalyptus oil, geraniol, carnosine, lipoic acid, propylene glycol, xanthan gum, hydroxyethyl acrylate/sodium acryloyl dimethyl taurate copolymer.

6. The composition according to any one of the preceding claims in a form suitable for topical administration.

7. A composition **characterized in that** it comprises the association of:
a) *Melaleuca alternifolia* oil extract,
b) mandelic acid,
c) α-Tocopherol polyethylene glycol succinate,
in the presence of at least an hygroscopic agent, wherein said three components of the association respectively are in weight ratio: tea tree oil/mandelic acid/Vit. E TPGS of 12/0.5/0.5 and the hygroscopic agent is a metal silicate between 1 and 10 % by total weigh of composition as defined in claims 1 to 6, for the use in the treatment of plantar hyperhidrosis as predisposing condition to cutaneous fungal infections of the foot and onychomycosis.

8. The composition for the use according to claim 7 wherein an effective amount of the composition is applied on skin and cutaneous annexes (nails) of the foot, once a day, generally in the evening before bedtime, after cleansing; and rub until completely absorbed.

## Patentansprüche

1. Zusammensetzung, **dadurch gekennzeichnet, dass** sie die Assoziation umfasst von:
a) Ölextrakt von *Melaleuca alternifolia,*
b) Mandelsäure,
c) α-Tocopherolpolyethylenglycolsuccinat,
in Gegenwart von mindestens einem hygroskopischen Mittel, wobei die drei Komponenten der Assoziation jeweils im Gewichtsverhältnis Teebaumöl/Mandelsäure/Vit.-E-TPGS von 12/0,5/0,5 sind und das hygroskopische Mittel ein Metallsilicat zwischen 1 und 10 % des Gesamtgewichtes der Zusammensetzung sind.

2. Zusammensetzung nach Anspruch 1, wobei das Metall Natrium, Lithium, Magnesium ist.

3. Zusammensetzung nach den Ansprüchen 1 und 2, wobei das hygroskopische Mittel Laponit ist.

4. Zusammensetzung nach einem der vorhergehenden Ansprüche, ferner umfassend: Trägermittel, Lösemittel, essentielle Öle, Duftstoffe und Parfüme, Konservierungsmittel, Emollientien und feuchtigkeitsspendende Mittel, Antioxidantien, Vitamine, Tenside, Suspendiermittel, Binder, Filmbildungs- und Viskositätsmittel, Verdicker, Chelatbildner, pharmazeutisch akzeptierte Puffermittel.

5. Zusammensetzung nach Anspruch 4, wobei Trägermittel, Lösemittel, essentielle Öle, Duftstoffe und Parfüme, Konservierungsmittel, Emollientien und feuchtigkeitsspendende Mittel, Antioxidationsmittel, Vitamine, Tenside, Suspendiermittel, Binder, Filmbildungs- und Viskositätsmittel, Verdicker, Chelatbildner, pharmazeutisch akzeptierte Puffermittel sind: Lavendelöl, Bergamottenöl, Eukalyptusöl, Geraniol, Carnosin, Liponsäure, Propylenglycol, Xanthangummi, Hydroxyethylacrylat/Natriumacryloyldimethyltaurat-Copolymer.

6. Zusammensetzung nach einem der vorhergehenden Ansprüche in einer Form, die zur topischen Verabreichung geeignet ist.

7. Zusammensetzung, **dadurch gekennzeichnet, dass** sie die Assoziation umfasst von:
a) Ölextrakt von *Melaleuca alternifolia,*
b) Mandelsäure,
c) α-Tocopherolpolyethylenglycolsuccinat,
in Gegenwart von mindestens einem hygroskopischen Mittel, wobei die drei Komponenten der Assoziation jeweils im Gewichtsverhältnis Teebaumöl/Mandelsäure/Vit.-E-TPGS von 12/0,5/0,5 sind und das hygroskopische Mittel ein Metallsilicat zwischen 1 und 10 % des Gesamtgewichtes der Zusammensetzung, wie in Anspruch 1 bis 6 definiert, ist, zur Benutzung bei der Behandlung von plantarer Hyperhidrose als prädisponierender Zustand für kutane Pilzinfektionen des Fußes und Onychomykose.

8. Zusammensetzung zur Benutzung nach Anspruch 7, wobei eine wirksame Menge der Zusammensetzung einmal täglich, im Allgemeinen am Abend vor der Schlafenszeit, nach Reinigung, auf Haut und kutane Annexen (Nägeln) des Fußes aufgebracht wird und eingerieben wird, bis sie vollständig absorbiert ist.

## Revendications

1. Composition **caractérisée en ce qu'**elle comprend l'association :
a) d'un extrait d'huile de *Melaleuca alternifolia,*
b) d'acide mandélique,
c) d'un succinate de polyéthylèneglycol α-tocophérol,
en présence d'au moins un agent hygroscopique, dans laquelle lesdits trois composants de l'association sont respectivement dans un rapport pondéral : huile de théier/acide mandélique/Vit. E TPGS de 12/0,5/0,5 et l'agent hygroscopique est un silicate métallique entre 1 et 10 % en poids total de la composition.

2. Composition selon la revendication 1, dans laquelle le métal est le sodium, le lithium, le magnésium.

3. Composition selon les revendications 1 et 2, dans laquelle l'agent hygroscopique est la laponite.

4. Composition selon l'une quelconque des revendications précédentes, comprenant en outre : des agents de support, des solvants, des huiles essentielles, des fragrances et des parfums, des conservateurs, des émollients et des agents hydratants, des antioxydants, des vitamines, des tensioactifs, des agents de mise en suspension, des liants, des agents filmogènes et de viscosité, des épaississants, des agents de chélation, des agents tampons acceptés en pharmacie.

5. Composition selon la revendication 4, dans laquelle les agents de support, les solvants, les huiles essentielles, les fragrances et les parfums, les conservateurs, les émollients et les agents hydratants, les agents antioxydants, les vitamines, les tensioactifs, les agents de mise en suspension, les liants, les agents filmogènes et de viscosité, les épaississants, les agents de chélation, les agents tampons acceptés en pharmacie, sont : l'huile de lavande, l'huile de bergamote, l'huile d'eucalyptus, le géraniol, la carnosine, l'acide lipoïque, le propylène glycol, la gomme de xanthane, un copolymère d'acrylate d'hydroxyéthyle/acryloyldiméthyltaurate de sodium.

6. Composition selon l'une quelconque des revendications précédentes sous une forme appropriée pour une administration topique.

7. Composition **caractérisée en ce qu'**elle comprend l'association :
a) d'un extrait d'huile de *Melaleuca alternifolia,*
b) d'acide mandélique,
c) d'un succinate de polyéthylèneglycol α-tocophérol,
en présence d'au moins un agent hygroscopique, dans laquelle lesdits trois composants de l'association sont respectivement dans un rapport pondéral : huile de théier/acide mandélique/Vit. E TPGS de 12/0,5/0,5 et l'agent hygroscopique est un silicate métallique entre 1 et 10 % en poids total de la composition telle que définie dans les revendications 1 à 6, pour utilisation dans le traitement de l'hyperhidrose plantaire en tant qu'affection prédisposant aux infections fongiques cutanées du pied et à une onychomycose.

8. Composition pour utilisation selon la revendication 7, dans laquelle une quantité efficace de la composition est appliquée sur la peau et les annexes cutanées (ongles) du pied, une fois par jour, généralement le soir avant le coucher, après nettoyage ; et frotter jusqu'à absorption complète.
